# EUROPÄISCHE PATENTSCHRIFT

(11) **EP 1 667 952 B1**
(45) Veröffentlichungstag und Bekanntmachung des Hinweises auf die Patenterteilung: **28.07.2010**
(21) Anmeldenummer: 04786994.6
(22) Anmeldetag: 23.09.2004
(51) Int. Cl.: C07C 45/27

(54) **VERFAHREN ZUR HERSTELLUNG EINES KETONS**
METHOD FOR PRODUCING A KETONE
PROCEDE DE PRODUCTION D'UNE CETONE

(30) Priorität: 25.09.2003 DE 10344595
(43) Veröffentlichungstag der Anmeldung: 14.06.2006
(73) Patentinhaber: BASF SE, 67056 Ludwigshafen (DE)
(72) Erfinder: TELES, Joaquim, H., 67166 Otterstadt (DE); RÖßLER, Beatrice, 67098 Bad Dürkheim (DE); PINKOS, Rolf, 67098 Bad Dürkheim (DE); GENGER, Thomas, 67245 Lambsheim (DE); PREISS, Thomas, 67256 Weisenheim am Sand (DE)
(86) Internationale Anmeldenummer: PCT/EP2004/010681
(87) Internationale Veröffentlichungsnummer: WO 2005/030690

(56) Entgegenhaltungen:
- EP-A- 1 035 119
- EP-A- 1 170 291
- GB-A- 649 680
- GB-A- 876 531
- US-A- 2 636 898
- PATENT ABSTRACTS OF JAPAN Bd. 2003, Nr. 12, 5. Dezember 2003 (2003-12-05) & JP 2004 059434 A (UBE IND LTD), 26. Februar 2004 (2004-02-26)
- ZAKHARKIN L I ET AL: "ISOMERIZATION OF TRANS-1,2-EPOXY-CIS,TRANS-5,9-CYCLODODECAD IENE, TRANS-1,2-EPOXY-TRANS,TRANS-5,9-CYCLODODEC ADIENE, AND TRANS-EPOXYCYCLODODECANE TO THE CORRESPONDING KETONES BY THE ACTIONOF LITHIUM AND MAGNESIUM IODIDES AND BROMIDES" JOURNAL OF ORGANIC CHEMISTRY OF THE USSR. (ZHURNAL ORGANICHESKOI KHIMII), CONSULTANTS BUREAU. NEW YORK, US, Bd. 26, Nr. 7, 1990, Seiten 1291-1294, XP000999526
- PATENT ABSTRACTS OF JAPAN Bd. 2002, Nr. 02, 2. April 2002 (2002-04-02) & JP 2001 302570 A (UBE IND LTD), 31. Oktober 2001 (2001-10-31)

## Beschreibung

Die vorliegende Erfindung betrifft ein Verfahren zur Herstellung eines Ketons, wobei 1,5,9-Cyclododecatrien durch Umsetzung mit Distickstoffmonoxid zu Cyclododecadienon oxidiert wird. Gemäß einer bevorzugten Ausführungsform wird in einem weiteren Schritt das Cyclododeca-4,8-dienon zu Cyclododecanon hydriert.

Cyclododecanon ist ein wichtiges Zwischenprodukt für die Herstellung von beispielsweise Laurinlactam, Dodecandicarbonsäure und daraus abgeleiteten Polyamiden wie beispielsweise Nylon 12 oder Nylon 6.12.

Cyclododecanon wird gemäß des gängigen technischen Verfahrens durch Luftoxidation von Cyclododecan in Anwesenheit von Borsäure zu Cyclododecylborat, Hydrolyse des Borates zu Cyclododecanol und anschließende Dehydrierung des Cyclododecanols hergestellt. Cyclododecan selbst wird weiterhin durch vollständige Hydrierung von Cyclododecatrien (CDT) gewonnen. Eine Beschreibung dieses technischen Verfahrens zur Synthese von Cyclododecanon findet sich unter anderem in T. Schiffer, G. Oenbrink, "Cyclododecanol, Cyclododecanon and Laurolactam" in Ullmann's Encyclopedia of Industrial Chemistry, 6th Edition, 2000, Electronic Release, Wiley VCH.

Das genannte technische Verfahren weist indessen eine Reihe von Nachteilen auf.

Zum einen gewährleistet die Oxidation von Cyclododecan mit Sauerstoff nur bei geringen Umsätzen eine akzeptable Selektivität. Auch unter Zusatz von Borsäure, die das entstandene Cyclododecanol in Form von Borsäureester vor weiterer Oxidation schützt, darf der Cyclododecanumsatz nicht über 30% liegen. Nach der Oxidation müssen die Borsäureester in einem getrennten Schritt hydrolysiert werden, und sowohl die Borsäure als auch das nicht umgesetzte Cyclododecan müssen in die Oxidation rückgeführt werden. Dabei entstehen auch Bor-haltige Abfälle, die schwer zu entsorgen sind. Als Hauptprodukte bilden sich dabei Cyclododecanol und Cyclododecanon im Verhältnis 10:1.

Zum anderen müssen das gebildete Gemisch aus Cyclododecanol und Cyclododecanon destillativ aufgetrennt und das Cyclododecanol durch Dehydrierung in Cyclododecanon überführt werden. Diese Dehydrierung ist endotherm und liefert ebenfalls nur einen partiellen Umsatz. Das nicht umgesetzte Cyclododecanol muss dann wiederum destillativ abgetrennt und in das Verfahren zurückgeführt werden.

Als Folge des nicht vollständigen Umsatzes beinhaltet das konventionelle Verfahren mehrere große Rückführströme und eine Reihe von technisch aufwändigen destillativen Trennungen.

Eine der der vorliegenden Erfindungen zugrunde liegenden Aufgaben war es daher auch, ein neues Verfahren zur Herstellung von Cyclododecanon bereitzustellen, das die Nachteile des aus dem Stand der Technik bekannten Verfahrens nicht aufweist.

Diese Aufgabe wurde erfindungsgemäß durch ein Verfahren zur Herstellung von Cyclododecanon gelöst, bei dem Cyclododecanon durch Hydrierung von Cyclododeca-4,8-dienon hergestellt wird gemäss Anspruch 9.

Im Rahmen dieser erfindungsgemäßen Lösung stellte sich das Problem, wie das als Edukt der Hydrierung eingesetzte Cyclododeca-4,8-dienon möglichst einfach und effektiv hergestellt werden kann.

Dieses Problem wurde erfindungsgemäß durch ein Verfahren gelöst, bei dem 1,5,9-Cyclododecatrien durch eine einstufige Umsetzung mit Distickstoffmonoxid zu Cyclododeca-4,8-dienon oxidiert wird.

Die Oxidation einer olefinischen Verbindung zu einem Aldehyd oder einem Keton mittels Distickstoffmonoxid ist beispielsweise in der GB 649,680 oder in der dazu äquivalenten US 2,636,898 beschrieben. Als cyclische olefinische Verbindungen werden dort allerdings lediglich Cyclopenten, Cyclohexen, Cycloocten und Cyclooctatetraen beschrieben. Cyclische Verbindungen mit mehr als 8 C-Atomen oder cyclische Verbindungen mit 3 C-C-Doppelbindungen werden dort nicht beschrieben.

In den neueren wissenschaftlichen Artikeln von G. L. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Allerdings beschränken sich die diesbezüglichen Offenbarungen ausschließlich auf Cyclopenten und Cyclohexen.

Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Herstellung von Cyclododeca-4,8-dienon aus 1,5,9-Cyclododecatrien war bislang lediglich über eine zweistufige Synthese möglich, bei der in einem ersten Schritt 1,5,9-Cyclododecatrien zu 1,2-Epoxycyclododeca-5,9-dien epoxidiert wurde und in einem zweiten Schritt das Epoxid katalytisch zu Cyclododeca-4,8-dienon umgelagert wurde. Dieses Verfahren ist beispielsweise in der US 3,063,986 und GB 876531 beschrieben. Dieses herkömmliche Verfahren weist zwei entscheidende Nachteile auf: Zum einen ist es schwierig, eine selektive Mono-Epoxidation zu erreichen. Zum anderen weist das Verfahren zwingend eine Zweistufigkeit auf.

EP 1 035 119 A1 offenbart ein Verfahren zur Herstellung von Epoxycyclododecadien. Es wird ein Verfahren zur Herstellung von 1,2-Epoxycyclododecadien durch Umsetzung von 1,5,9-Cyclododecatrien mit Wasserstoffperoxid in Gegenwart von Carbonsäuren offenbart.

EP 1 170 291 A1 offenbart ein Verfahren zur Epoxidation von Cyclododecatrien durch Umsetzung mit Wasserstoffperoxid in Gegenwart von Katalysatoren. Die Katalysatoren umfassen eine Wolframverbindung und Mineralsäuren.

L. I. Zakharkin et al. im Journal of Organic Chemistry of the USSR, Band 26, Nr. 7, 1990, Seiten 1291-1294 offenbart ein Verfahren zur Isomerisierung von Epoxycyclododecadienen zu den entsprechenden Ketonen. Dabei wird auch die Synthese des Epoxids durch Epoxidierung von Cyclododecatrien mit Hydroperoxiden beschrieben. Die Umlagerung findet in Gegenwart eines Katalysators statt.

JP 2001302570 (Patent Abstracts) offenbart ein Verfahren zur Herstellung von Cyclododecanon und Cyclododecanol durch Hydrierung von 1,2-Epoxycyclododecadien. Dabei wird zunächst eine Hydrierung von 1,2-Epoxy-5,9-cyclododecadien in Gegenwart eines Katalysators durchgeführt und anschließend eine Umlagerung des Epoxids zum Keton.

Demgegenüber bietet die oben beschriebene, im Rahmen des erfindungsgemäßen Verfahrens bevorzugte Ausführungsform die Möglichkeit, ausgehend von 1,5,9-Cyclododecatrien in einem einzigen Schritt bei hoher Selektivität zu Cyclododeca-4,8-dienon zu gelangen.

Das erfindungsgemäße Gesamtverfahren zur Herstellung von Cyclododecanon umfasst demgemäß zwei Schritte, wobei in einem ersten Schritt 1,5,9-Cyclododecatrien mit Distickstoffmonoxid unter Erhalt von Cyclododeca-4,8-dienon umgesetzt wird und in einem zweiten Schritt das Cyclododeca-4,8-dienon unter Erhalt von Cyclododecanon hydriert wird.

Gegenüber dem oben beschriebenen gängigen Verfahren, das zwingend die vier Schritte
- Vollständige Hydrierung von 1,5,9-Cyclododecatrien zu Cyclododecan;
- Luftoxidation des Cyclododecans in Anwesenheit von Borsäure zu Cyclododecylborat;
- Hydrolyse des Borates zu Cyclododecanol;
- Dehydrierung des Cyclododecanols unter Erhalt von Cyclododecanon
umfasst, zeichnet sich das erfindungsgemäße Verfahren zur Herstellung von Cyclododecanon demgemäß unter anderem dadurch aus, dass ausgehend vom gleichen Edukt, 1,5,9-Cyclododecatrien, das Produkt Cyclododecanon unter Einsparung von zwei Reaktionsstufen hergestellt werden kann und die Anzahl der Reaktionsstufen damit halbiert wird.

Demgemäß betrifft die vorliegende Erfindung ein wie oben beschriebenes Verfahren zur Herstellung von Cyclododecanon, umfassend die Schritte (I) und (II)
(I) Umsetzung von 1,5,9-Cyclododecatrien mit Distickstoffmonoxid unter Erhalt von Cyclododeca-4,8-dienon;
(II) Hydrierung des gemäß (I) erhaltenen Cyclododeca-4,8-dienons unter Erhalt von Cyclododecanon.

Ebenso betrifft die vorliegende Erfindung ein Verfahren zur Herstellung eines Ketons, wobei 1,5,9-Cyclododecatrien mit Distickstoffmonoxid unter Erhalt von Cyclododeca-4,8-dienon umgesetzt wird.

Das zur Umsetzung verwendete Distickstoffmonoxid kann grundsätzlich in reiner Form oder in Form eines geeigneten Gasgemisches, enthaltend Distickstoffmonoxid, eingesetzt werden. Dabei kann das Distickstoffmonoxid weiterhin grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig oder überkritisch, vorzugsweise flüssig, und wird im Rahmen der vorliegenden Erfindung weiter als Gasgemisch bezeichnet.

Wird ein Gasgemisch eingesetzt, so ist dessen Gehalt an Distickstoffmonoxid im Wesentlichen beliebig, solange gewährleistet ist, dass die erfindungsgemäße Umsetzung möglich ist.

Gemäß einer bevorzugten Ausführungsform des erfindungsgemäßen Verfahrens wird ein mindestens 10 Vol.-% Distickstoffmonoxid enthaltendes Gasgemisch eingesetzt, wobei wiederum bevorzugt Gemische mit einem Distickstoffmonoxid-Gehalt im Bereich von 20 bis 99,9 Vol.-%, weiter bevorzugt im Bereich von 40 bis 99,5 Vol.-%, weiter bevorzugt im Bereich von 60 bis 99,5 Vol.-% und insbesondere bevorzugt im Bereich von 80 bis 99,5 Vol.-% eingesetzt werden.

Im Rahmen der vorliegenden Erfindung wird die Zusammensetzung der Gasgemische in Vol.-% angegeben. Dabei beziehen sich die Angaben auf die Zusammensetzung der Gasgemische bei Umgebungsdruck und Umgebungstemperatur.

Demgemäß betrifft die vorliegende Erfindung auch ein wie oben beschriebenes Verfahren, das dadurch gekennzeichnet ist, dass Cyclododecatrien mit einem Gasgemisch, enthaltend 20 bis 99,9 Gew.-% Distickstoffmonoxid, bezogen auf das Gesamtgewicht des Gasgemisches, umgesetzt wird.

Der Begriff "Gasgemisch", wie er im Rahmen der vorliegenden Erfindung verwendet wird, bezeichnet weiterhin Gemische, die neben Distickstoffmonoxid noch mindestens eine weitere Komponente, vorzugsweise ein weiteres Gas, enthalten. Dabei kann es sich bei der Komponente auch um ein Gas handeln, das bei den gewählten Bedingungen beispielsweise flüssig vorliegt. Hierbei sind im Wesentlichen sämtliche Gase denkbar, solange gewährleistet ist, dass die Umsetzung von Cyclododecatrien mit Distickstoffmonoxid möglich ist. Insbesondere sind demzufolge Gasgemische bevorzugt, die neben Distickstoffmonoxid mindestens ein Inertgas enthalten. Der Begriff "Inertgas", wie er im Rahmen der vorliegenden Erfindung verwendet wird, bezeichnet ein Gas, das sich hinsichtlich der Umsetzung von Distickstoffmonoxid mit Cyclododecatrien inert verhält. Als Inertgase sind beispielsweise Stickstoff, Kohlendioxid, Kohlenmonoxid, Wasserstoff, Wasser, Argon, Methan, Ethan und Propan zu nennen.

Ebenso können im Gasgemisch auch Komponenten, vorzugsweise Gase enthalten sein, die sich bei der Umsetzung von N₂O mit Cyclododecatrien nicht als Inerte, vorzugsweise Inertgase verhalten. Als solche Gase sind unter anderem NOₓ oder beispielsweise Sauerstoff zu nennen. Der Begriff "NOₓ", wie er im Rahmen der vorliegenden Erfindung verwendet wird, bezeichnet sämtliche Verbindungen NₐO_{b} außer Distickstoffmonoxid, wobei a 1 oder 2 ist und b eine Zahl von 1 bis 6. Statt dem Begriff "NOₓ" wird im Rahmen der vorliegenden. Erfindung auch der Begriff "Stickoxide" verwendet. In einem solchen Fall ist es bevorzugt, solche Gasgemische einzusetzen, deren Gehalt an diesen Gasen im Bereich von 0 bis 0,5 Vol.-%, bezogen auf das Gesamtvolumen des Gasgemisches, liegt.

Demgemäß beschreibt die vorliegende Erfindung auch ein wie oben beschriebenes Verfahren, das dadurch gekennzeichnet ist, dass das Gasgemisch 0 bis 0,5 Vol.-% Sauerstoff oder 0 bis 0,5 Vol.-% Stickoxide oder sowohl 0 bis 0,5 Vol.-% Sauerstoff als auch 0 bis 0,5 Vol.-% Stickoxide, jeweils bezogen auf das Gesamtvolumen des Gasgemisches, enthält. Ein Wert von beispielsweise 0,5 Vol.-% bezeichnet hierbei einen Gesamtgehalt aller möglichen Stickoxide außer Distickstoffmonoxid von 0,5 Vol.-%.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Erfindungsgemäß kann Distickstoffmonoxid oder das Gasgemisch enthaltend Distickstoffmonoxid in jeder Form eingesetzt werden, insbesondere gasförmig oder auch in flüssiger Form. Dabei kann Distickstoffmonoxid oder das Gasgemisch enthaltend Distickstoffmonoxid mit allen den Fachmann bekannten Verfahren verflüssigt werden, vorzugsweise durch geeignete Wahl des Drucks und der Temperatur.

Erfindungsgemäß ist es auch möglich, dass Distickstoffmonoxid oder das Gasgemisch enthaltend Distickstoffmonoxid zunächst in einem geeigneten Lösungsmittel absorbiert wird und so der Reaktion zugesetzt wird.

Gemäß einer bevorzugten Ausführungsform der vorliegenden Erfindung ist die Distickstoffmonoxidquelle mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Distickstoffmonoxidquelle dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Distickstoffmonoxidquelle dienen.

Demgemäß betrifft die vorliegende Erfindung auch ein wie oben beschriebenes Verfahren, das dadurch gekennzeichnet ist, dass als Distickstoffmonoxidquelle mindestens ein Distickstoffmonoxid enthaltendes Abgas mindestens eines industriellen Verfahrens dient.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in der US 3,656,899 beschrieben ist, deren diesbezüglicher Inhalt durch Bezugnahme in den Kontext der vorliegenden Anmeldung vollumfänglich aufgenommen wird. Ebenfalls bevorzugt wird weiter die Herstellung über die katalytische Oxidation von Ammoniak, wie dies beispielsweise in der US 5,849,257 oder in der WO 98/25698 beschrieben ist, deren diesbezüglicher Inhalt durch Bezugnahme in den Kontext der vorliegenden Anmeldung vollumfänglich aufgenommen wird.

Der Begriff "Distickstoffmonoxidquelle" bezeichnet im Rahmen der vorliegende Erfindung sowohl Ausführungsformen, in denen das genannte Abgas in unmodifizierter Form in der erfindungsgemäßen Umsetzung von Cyclododecatrien eingesetzt wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Abgases verändert wird. Demgemäß umfasst der Begriff "Modifikation" unter anderem Ausführungsformen, in denen ein Distickstoffmonoxid enthaltendes Abgas bezüglich des Distickstoffmonoxidgehaltes gemäß mindestens eines geeigneten Verfahrens aufkonzentriert wird. Solche Verfahren sind beispielsweise in der DE-OS 27 32 267, der EP 1 076 217 A2 oder der WO 00/73202 A1 beschrieben, deren diesbezüglicher Inhalt durch Bezugnahme in den Kontext der vorliegenden Anmeldung vollumfänglich einbezogen wird.

Das Gasgemisch kann dabei im Rahmen der vorliegenden Erfindung auch einer Modifikation unterworfen werden, um die Konzentration von inerten und störenden Verbindungen im Gasgemisch zu reduzieren.

Diese Modifikation kann erfindungsgemäß beispielsweise eine Absorption des Gasgemisches in einem geeigneten Lösungsmittel und anschließende Desorption umfassen, um inerte Verbindungen aus dem Gasgemisch zu entfernen. Ein geeignetes Lösungsmittel für die Absorption ist beispielsweise Wasser, wie in der DT 20 40 219 beschrieben wird.

Erfindungsgemäß kann die Modifikation des Gasgemisches auch einen weiteren Reinigungsschritt, beispielsweise einen Schritt zur Abtrennung von NOₓ aus dem Gasgemisch umfassen. Derartige Verfahren zur Abtrennung von NOₓ sind grundsätzlich aus dem Stand der Technik bekannt. Erfindungsgemäß können alle dem Fachmann bekannten Verfahren zur Abtrennung von NOₓ eingesetzt werden.
Erfindungsgemäß ist es dabei bevorzugt, dass das Abgas einer Behandlung umfassend eine Absorption in einem geeigneten Lösungsmittel und anschließende Desorption unterworfen wird, um inerte Verbindungen zu entfernen. Ein geeignetes Lösungsmittel ist beispielsweise Wasser, wie in der DT 20 40 219 beschrieben.

Gemäß einer beispielsweise bevorzugten Ausführungsform des erfindungsgemäßen Verfahrens ist es zur Aufkonzentrierung möglich, das oben genannte, Distickstoffmonoxid enthaltende Abgas mindestens einer Adsorptionskolonne zuzuführen und das Distickstoffmonoxid in mindestens einem organischen Lösungsmittel einzulösen. Als Lösungsmittel ist hierfür beispielsweise Cyclododecatrien geeignet. Diese erfindungsgemäße Verfahrensvariante bietet den Vorteil, dass die resultierende Lösung von Distickstoffmonoxid in Cyclododecatrien ohne weitere Aufarbeitung der erfindungsgemäßen Umsetzung zugeführt werden kann. Diese Lösung von Distickstoffmonoxid in Cyclododecatrien kann Distickstoffmonoxid in sämtlichen denkbaren Konzentrationen bis hin zur Sättigung enthalten. Gemäß anderer Ausführungsformen kann mindestens ein weiteres Lösungsmittel oder ein Gemisch aus Cyclododecatrien und mindestens einem weiteren Lösungsmittel zur Adsorption verwendet werden. Solche weiteren Lösungsmittel sind beispielsweise alle geeigneten gängigen organischen Lösungsmittel. Bevorzugte Lösungsmittel sind unter anderem N-Methylpyrrolidon, Dimethylformamid, Dimethylsulfoxid, Propylencarbonat, Sulfolan oder N,N-Dimethylacetamid. Wird mindestens ein weiteres Lösungsmittel oder ein Gemisch aus Cyclododecatrien und mindestens einem weiteren Lösungsmittel eingesetzt, so wird gemäß einer weiter bevorzugten Ausführungsform aus der mit Distickstoffmonoxid angereicherten Lösung in mindestens einem geeigneten Desorptionsschritt das Distickstoffmonoxid zumindest teilweise, bevorzugt im wesentlichen vollständig gewonnen und der erfindungsgemäßen Umsetzung zugeführt.

Gemäß einer weiteren Ausführungsform kann die chemische Zusammensetzung eines Abgases auch durch Zusatz von reinem Distickstoffmonoxid zu dem Abgas verändert werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage oder einer Hydroxylaminanlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandisäureanlage handelt es sich um den im Wesentlichen identischen Anlagentyp.

Eine beispielsweise typische Zusammensetzung eines Abgases einer Adipinsäureanlage oder einer Dodecandisäureanlage ist in der folgenden Tabelle wiedergegeben:

| Komponente | Konzentrationen / Gew.-% |
|---|---|
| NOₓ | 19 - 25 |
| N₂O | 20 - 28 |
| N₂ | 30 - 40 |
| O₂ | 7 - 10 |
| CO₂ | 2 - 3 |
| H₂O | ~ 7 |

Der Abgasstrom einer Adipinsäureanlage oder einer Dodecandisäureanlage kann direkt in dem erfindungsgemäßen Verfahren eingesetzt werden. Bevorzugt wird der Abgasstrom der Adipinsäureanlage oder einer Dodecandisäureanlage vor dem Einsatz zur Umsetzung des Cyclododecatriens gereinigt. Dabei wird beispielsweise vorteilhaft der Gehalt des Abgasstroms an Sauerstoff und/oder Stickoxiden auf Gehalte im Bereich von jeweils 0 bis 0,5 Vol.-% eingestellt. In der oben zitierten Schrift von A. K. Uriarte et al. werden verschiedene Möglichkeiten offenbart, wie ein solcher Abgasstrom zur Verwendung bei der katalytischen Benzolhydroxylierung gereinigt werden kann. Es werden Absorptionsverfahren wie beispielsweise Druckwechselabsorption, Membran-Trennverfahren, Tieftemperaturdestillation oder eine Kombination aus selektiver katalytischer Reduktion mit Ammoniak, gefolgt von der katalytischen Entfernung von Sauerstoff beschrieben. Sämtliche dieser Reinigungsmethoden sind auch anwendbar, um den Distickstoffmonoxid enthaltenden Abgasstrom einer industriellen Anlage wie beispielsweise einer Adipinsäureanlage oder einer Dodecandisäureanlage oder einer Salpetersäureanlage zu reinigen. Ganz besonders bevorzugt sind die destillative Reinigung und damit die destillative Aufkonzentrierung des Abgasstroms einer Adipinsäureanlage oder einer Dodecandisäureanlage oder einer Salpetersäureanlage.

Besonders bevorzugt wird im Rahmen der vorliegenden Erfindung der Abgasstrom einer Adipinsäureanlage oder einer Dodecandisäureanlage im Falle, dass dieser Sauerstoff und/oder Stickoxide zu jeweils mehr als 0,5 Vol.-% enthält, aufgereinigt.

Demgemäß beschreibt die vorliegende Erfindung auch ein wie oben beschriebenes Verfahren, das dadurch gekennzeichnet ist, dass zur Umsetzung des Cyclododecatriens der Abgasstrom einer Adipinsäureanlage oder einer Dodecandisäureanlage eingesetzt wird.

Weiter beschreibt die vorliegende Erfindung demgemäß ein wie oben beschriebenes Verfahren, das dadurch gekennzeichnet ist, dass der gegebenenfalls bevorzugt destillativ gereinigte Abgasstrom der Adipinsäureanlage oder einer Dodecandisäureanlage Sauerstoff und/oder Stickoxide im Bereich von jeweils 0 bis 0,5 Vol.-% enthält.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandisäureanlage gespeist werden. Ebenso ist es möglich, eine derartige Salpetersäureanlage allein durch Abgase einer Adipinsäureanlage und/oder durch Abgase einer Dodecandisäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NOₓ | < 0,1 |
| N₂O | 8 - 36 |
| N₂ | 57 - 86 |
| O₂ | 3 - 9 |
| CO₂ | 1 - 4 |
| H₂O | ~ 0,6 |

Der Abgasstrom einer Salpetersäureanlage kann direkt in dem erfindungsgemäßen Verfahren eingesetzt werden: Bevorzugt wird der Abgasstrom der Salpetersäureanlage vor dem Einsatz zur Umsetzung des Cyclododecatriens gereinigt. Dabei wird beispielsweise vorteilhaft der Gehalt des Abgasstroms an Sauerstoff und/oder Stickoxiden auf Gehalte im Bereich von jeweils 0 bis 0,5 Vol.-% eingestellt. Geeignete Verfahren, mit denen diese Werte einstellbar sind, sind obenstehend im Rahmen der Adipinsäureanlage und Dodecandisäureanlage beschrieben. Ganz besonders bevorzugt sind auch im Rahmen der Abgase der Salpetersäureanlage die destillative Reinigung und damit die destillative Aufkonzentrierung.

Besonders bevorzugt wird im Rahmen der vorliegenden Erfindung der Abgasstrom einer Salpetersäureanlage im Falle, dass dieser Sauerstoff und/oder Stickoxide zu jeweils mehr als 0,5 Vol.-% enthält, aufgereinigt.

Demgemäß beschreibt die vorliegende Erfindung auch ein wie oben beschriebenes Verfahren, das dadurch gekennzeichnet ist, dass zur Umsetzung des Cyclododecatriens der Distickstoffmonoxid enthaltende Abgasstrom einer Salpetersäureanlage eingesetzt wird.

Weiter beschreibt die vorliegende Erfindung demgemäß ein wie oben beschriebenes Verfahren, das dadurch gekennzeichnet ist, dass der gegebenenfalls bevorzugt destillativ gereinigte Abgasstrom der Salpetersäureanlage Sauerstoff und/oder Stickoxide in einem Bereich von 0 bis 0,5 Vol.-% enthält.

Gemäß einer ebenfalls bevorzugten Ausführungsform des erfindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Umsetzung eingesetzt werden. Ebenso ist es möglich, diesen Strom bezüglich des Distickstoffmonoxidgehaltes, wie oben beschrieben, geeignet aufzukonzentrieren.

Demgemäß betrifft die vorliegende Erfindung auch ein wie oben beschriebenes Verfahren, das dadurch gekennzeichnet ist, dass die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

Ebenso beschreibt die vorliegende Erfindung auch ein integriertes Verfahren zur Herstellung von Cyclododeca-4,8-dienon, das mindestens die folgenden Schritte (i) und (ii) umfasst:
(i) Bereitstellung eines Distickstoffmonoxid enthaltenden Gasgemisches, enthaltend jeweils von 0 bis 0,5 Vol.-% Sauerstoff und/oder Stickoxide, auf der Basis mindestens eines Abgasstroms mindestens einer Adipinsäureanlage und/oder mindestens einer Dodecandisäureanlage und/oder mindestens einer Hydroxylaminanlage und/oder mindestens einer mit dem Abgas einer Adipinsäureanlage und/oder einer Dodecandisäureanlage und/oder einer Hydroxylaminanlage betriebenen Salpetersäureanlage;
(ii) Umsetzung von 1,5,9-Cyclododecatrien mit dem gemäß (i) bereitgestellten Gasgemisch unter Erhalt von Cyclododeca-4,8-dienon.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in der US 3,656,899 beschrieben ist, deren diesbezüglicher Inhalt durch Bezugnahme in den Kontext der vorliegenden Anmeldung vollumfänglich aufgenommen wird. Ebenfalls bevorzugt wird weiter die Herstellung über die katalytische Oxidation von Ammoniak, wie dies beispielsweise in der US 5,849,257 oder in der WO 98/25698 beschrieben ist, deren diesbezüglicher Inhalt durch Bezugnahme in den Kontext der vorliegenden Anmeldung vollumfänglich aufgenommen wird.

Im Rahmen der erfindungsgemäßen Umsetzung des 1,5,9-Cyclododecatriens mit Distickstoffmonoxid kann mindestens ein geeignetes Lösungsmittel oder Verdünnungsmittel eingesetzt werden. Als solche sind unter anderem Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung noch eine C-C-Dreifachbindung noch eine Aldehydgruppe aufweisen.

Im Allgemeinen ist bei der erfindungsgemäßen Umsetzung mit Distickstoffmonoxid der Zusatz eines Lösungsmittels oder Verdünnungsmittel nicht notwendig.

Generell existieren hinsichtlich der Reaktionsbedingungen der Umsetzung des 1,5,9-Cyclododecatriens keine besonderen Beschränkungen, solange gewährleistet ist, dass aus der Umsetzung Cyclododeca-4,8-dienon erhalten wird.

Die Temperaturen bei der Umsetzung von 1,5,9-Cyclododecatrien mit Distickstoffmonoxid liegen bevorzugt im Bereich von 140 bis 350 °C, weiter bevorzugt im Bereich von 160 bis 275 °C oder in einem Bereich von 200 bis 310 °C und besonders bevorzugt im Bereich von 180 bis 250 °C oder von 250 bis 300 °C.

Im Rahmen des erfindungsgemäßen Verfahrens ist es möglich, die Reaktion bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung von 1,5,9-Cyclododecatrien mit Distickstoffmonoxid liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 300 bar und besonders bevorzugt im Bereich von 50 bis 200 bar.

Im Rahmen des erfindungsgemäßen Verfahrens ist es möglich, die Reaktion bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Demgemäß betrifft die vorliegende Erfindung auch ein wie oben beschriebenes Verfahren, das dadurch gekennzeichnet ist, dass die Umsetzung gemäß (ii) bei einer Temperatur im Bereich von 140 bis 350 °C und einem Druck im Bereich von 1 bis 1000 bar durchgeführt wird.

Hinsichtlich der für die Umsetzung einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Gemäß einer bevorzugten Ausführungsform wird die erfindungsgemäße Umsetzung mit Distickstoffmonoxid in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit 1,5,9-Cyclododecatrien zugeführt wird, liegt das Molverhältnis von Distickstoffmonoxid zu 1,5,9-Cyclododecatrien im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4. Gemäß einer alternativen Ausführungsform liegen bevorzugte Bereiche bei 0,2 bis 4, bevorzugt im Bereich von 0,3 bis 3, weiter bevorzugt im Bereich von 0,4 bis 2 und besonders bevorzugt im Bereich von 0,4 bis 1,5.

Gemäß einer besonders bevorzugten Ausführungsform wird das erfindungsgemäße Verfahren so durchgeführt, dass bei einer sehr hohen Selektivität bezüglich Cyclododeca-4,8-dienon ein Umsatz von 1,5,9-Cyclododecatrien im Bereich von bis zu 95 %, bevorzugt im Bereich von 1 bis 80%, weiter bevorzugt von 5 bis 50%, besonders bevorzugt im Bereich von 8 bis 25 %. Gemäß einer alternativen Ausführungsform liegen bevorzugte Bereiche bei 10 bis 80 %, weiter bevorzugt im Bereich von 21 bis 75 % und insbesondere bevorzugt im Bereich von 20 bis 50 % erreicht wird. Dabei liegt die Selektivität, bezogen auf Cyclododeca-4,8-dienon, im Allgemeinen bei mindestens 90 %, bevorzugt bei mindestens 92,5 % und besonders bevorzugt bei mindestens 95 %.

Demgemäß betrifft die vorliegende Erfindung auch ein wie oben beschriebenes Verfahren, das dadurch gekennzeichnet ist, dass die Umsetzung von Distickstoffmonoxid mit 1,5,9-Cyclododecatrien bei eine Selektivität, bezogen auf Cyclododeca-4,8-dienon, von mindestens 90 % einen Umsatz von 1,5,9-Cyclododecatrien im Bereich von 1 bis 80 %, bevorzugt im Bereich von 5 bis 30 % aufweist.

Im Rahmen der vorliegenden Erfindung kann grundsätzliches jedes 1,5,9-Cyclododecatrien oder jedes Gemisch aus zwei und mehr unterschiedlichen 1,5,9-Cyclododecatrienen mit Distickstoffmonoxid umgesetzt werden. Unter anderem sind hierbei beispielsweise is 5,9-Cyclododecatriene beispielsweise cis,trans,trans-1,5,9-Cyclododecatrien oder cis,cis,trans-1,5,9-Cyclododecatrien oder all-trans-1,5,9-Cyclododecatrien, zu nennen.

Gemäß einer ganz besonders bevorzugten Ausführungsform des erfindungsgemäßen Verfahrens wird als 1,5,9-Cyclododecatrien cis,trans,trans-1,5,9-Cyclododecatrien eingesetzt.

Demgemäß betrifft die vorliegende Erfindung auch ein wie oben beschriebenes Verfahren, das dadurch gekennzeichnet ist, dass als 1,5,9-Cyclododecatrien cis,trans,trans-1,5,9-Cyclododecatrien eingesetzt wird.

Insbesondere beschreibt die vorliegende Erfindung daher auch ein wie oben beschriebenes Verfahren, das dadurch gekennzeichnet ist, dass cis,trans,trans-1,5,9-Cyclododecatrien mit Distickstoffmonoxid unter Erhalt von Cyclododeca-4,8-dienon umgesetzt wird.

Im allgemeinen resultiert aus der erfindungsgemäßen Umsetzung von cis,trans,trans-1,5,9-Cyclododecatrien mit Distickstoffmonoxid ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon enthält. Bevorzugt wird erfindungsgemäß ein Isomerengemisch erhalten, bei dem trans,cis-Isomer und cis,trans-Isomer in etwa in gleichen Mengen gebildet werden und das trans,trans-Isomer im Vergleich zu den beiden anderen Isomeren in nur geringen Mengen gebildet wird. Ein beispielsweise typisches Isomerengemisch weist demgemäß die Isomeren in molaren Verhältnissen von in etwa 1 : 1 : 0,08 auf.

Die erfindungsgemäße Umsetzung mindestens eines 1,5,9-Cyclododecatriens und insbesondere bevorzugt die Umsetzung von cis,trans,trans-1,5,9-Cyclododecatrien kann grundsätzlich in Anwesenheit eines Katalysators erfolgen. Gemäß einer besonders bevorzugten Ausführungsform des erfindungsgemäßen Verfahrens wird die Umsetzung mit Distickstoffmonoxid ohne Zusatz eines Katalysators durchgeführt.

Demgemäß beschreibt die vorliegende Erfindung auch ein wie oben beschriebenes Verfahren, das dadurch gekennzeichnet ist, dass die Umsetzung von 1,5,9-Cyclododecatrien mit Distickstoffmonoxid ohne Zusatz eines Katalysators durchgeführt wird.

Im Allgemeinen ist bei der erfindungsgemäßen Umsetzung mit Distickstoffmonoxid der Zusatz eines Lösungsmittels oder Verdünnungsmittel nicht notwendig.

Das eingesetzte 1,5,9-Cyclododecatrien kann beispielsweise durch Trimerisierung von reinem 1,3-Butadien hergestellt werden, wie dies beispielsweise in T. Schiffer, G. Oenbrink, "Cyclodecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei der Trimerisierung in Anwesenheit von Ziegler-Katalysatoren cis,trans;trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Während sämtliche dieser Cyclododecatriene im Rahmen des erfindungsgemäßen Verfahrens mittels Distickstoffmonoxid einzeln oder als Gemisch aus zwei oder mehr davon oxidiert werden können, ist, wie oben beschrieben, die Umsetzung von cis,trans,trans-1,5,9-Cyclododecatrien im Rahmen des vorliegenden erfindungsgemäßen Verfahrens besonders bevorzugt. Dieses cis,trans,trans-1,5,9-Cyclododecatrien wird besonders bevorzugt gemäß des in dem oben erwähnten Artikel von Weber et al. hergestellt, dessen diesbezüglicher Inhalt durch Bezugnahme in den Kontext der vorliegenden Anmeldung vollumfänglich einbezogen wird.

Demgemäß beschreibt die vorliegende Erfindung auch ein wie oben beschriebenes Verfahren, das dadurch gekennzeichnet ist, dass das als Edukt eingesetzte 1,5,9-Cyclododecatrien durch Trimerisierung von 1,3-Butadien unter Verwendung eines Titan-Katalysators hergestellt wird.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders bevorzugt.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser bevorzugten Trimerisierung werden im Allgemeinen Gemische erhalten, die mindestens 95 Gew.-%, bevorzugt mindestens 96 Gew.-% und weitere bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die Umsetzung mit Distickstoffmonoxid verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung mit Distickstoffmonoxid einzusetzen. Bevorzugt findet eine solche Aufreinigung im Rahmen des erfindungsgemäßen Verfahrens nicht statt.

Hinsichtlich weiterer Details zur Trimerisierung sei auf den Artikel von Weber et al. verwiesen.

Aus der erfindungsgemäßen Umsetzung von 1,5,9-Cyclododecatrien mit Distickstoffmonoxid wird im Allgemeinen ein Gemisch erhalten, das Cyclododeca-4,8-dienon, und gegebenenfalls nicht umgesetztes Edukt und/oder gegebenenfalls mindestens ein Nebenprodukt enthält. Je nach weiterer Verwertung und/oder Aufarbeitung kann aus diesem Gemisch das das Cyclododeca-4,8-dienon abgetrennt werden. Im Falle, dass das Gemisch Cyclododeca-4,8-dienon und beispielsweise ein Diketon wie Cyclododecendion enthält, ist es möglich, das das Cyclododeca-4,8-dienon, in einfacher Weise abzutrennen und einem weiteren Verfahrensschritt wie beispielsweise der Partialhydrierung zu Cyclododecenon oder bevorzugt der Hydrierung zu Cyclododecanon zuzuführen. Aus diesem Gemisch kann das Cyclododeca-4,8-dienon durch mindestens eine geeignete Methode abgetrennt werden. Bevorzugt ist hierbei die destillative Abtrennung. Die Destillation erfolgt hierbei bei einem Druck im Bereich von im Allgemeinen 0,001 bis 2 bar, bevorzugt im Bereich von 0,01 bis 1 bar und insbesondere bevorzugt im Bereich von 0,03 bis 0,5 bar, beispielsweise von 0,04 bis 0,5 bar oder 0,05 bis 0,5 bar.

Das erfindungsgemäß aus der Umsetzung von 1,5,9-Cyclododecatrien mit Distickstoffmonoxid erhaltene Cyclododeca-4,8-dienon, kann einem oder mehreren beliebigen weiteren Verfahren zugeführt werden. Beispielsweise kann die Ketogruppe des Cyclododeca-4,8-dienons einer chemischen Reaktion unterworfen werden. Ebenso kann mindestens eine der C-C-Doppelbindungen einer chemischen Reaktion unterworfen werden. Beispielsweise bevorzugt können mindestens eine C-C-Doppelbindung, bevorzugt beide C-C-Doppelbindungen hydriert werden.

Unabhängig davon, welches Regioisomere von Cyclododeca-4,8-dienon oder welches Gemisch aus mindestens zwei regioisomeren Cyclododeca-4,8-dienonen aus der erfindungsgemäßen Umsetzung mit Distickstoffmonoxid erhalten wird, wird dieses Regioisomere oder dieses Regioisomerengemisch bevorzugt zu Cyclododecanon hydriert.

Gemäß einer besonders bevorzugten Ausführungsform des erfindungsgemäßen Verfahrens wird Cyclododeca-4,8-dienon zu Cyclododecanon hydriert.

Demgemäß betrifft die vorliegende Erfindung auch ein wie oben beschriebenes Verfahren, das dadurch gekennzeichnet ist, dass das aus der Umsetzung von 1,5,9-Cyclododecatrien mit Distickstoffmonoxid erhaltene Cyclododeca-4,8-dienon unter Erhalt von Cyclododecanon hydriert wird.

Für die Hydrierung des Cyclododeca-4,8-dienons können sämtliche geeigneten Katalysatoren eingesetzt werden. Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß eingesetzten Katalysatoren Pd.

Im erfindungsgemäßen Verfahren bevorzugt eingesetzte homogene Katalysatoren enthalten mindestens ein Element der 8., 9. oder 10. Nebengruppe. Weiter bevorzugt sind homogenen Katalysatoren, die Ru, Rh, Ir und/oder Ni enthalten. Beispielsweise sind hierbei etwa RhCI(TTP)₃ oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind, deren diesbezügliche Offenbarung in den Kontext der vorliegenden Anmeldung vollumfänglich einbezogen wird. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH oder (TPP)₃(CO)RuCl₂.

Insbesondere bevorzugt wird im Rahmen des erfindungsgemäßen Verfahrens mindestens ein heterogener Katalysator eingesetzt, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die im erfindungsgemäßen Verfahren eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salze oder Carbonaten ausfällt, die erhaltenen Niederschläge anschließend trocknet und diese dann durch Calcinierung bei im allgemeinen 300 bis 700 °C, insbesondere 400 bis 600 °C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 - 700 °C, insbesondere 100 bis 400 °C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytischhydrierend wirkende Komponente beispielsweise durch Imprägnierung auf ein Trägermaterial aufgebracht worden sind.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600 °C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817 oder EP 0 285 420 A1 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbaren Katalysatoren dienen.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung mit mindestens einem Suspensionskatalysator durchgeführt, so wird bevorzugt in mindestens einem Rührreaktor oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihrem Volumen und/oder ihrem Querschnitt und/oder durch die Hydrierbedingungen in den Reaktoren unterscheiden.

Wird beispielsweise im Rahmen des erfindungsgemäßen Verfahrens die Hydrierung mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Gemäß einer bevorzugten Ausführungsform des erfindungsgemäßen Verfahrens wird der für die Hydrierung eingesetzte mindestens eine Katalysator aus dem Produktgemisch der Hydrierung abgetrennt. Diese Abtrennung kann in Abhängigkeit vom eingesetzten Katalysator gemäß jeder geeigneten Verfahrensführung erfolgen.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurückzugewinnen.

Wird als Katalysator bei der Hydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurückzugewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärmeabfuhr kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabführung integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung an mindestens einem suspendierten Katalysator durchgeführt, liegt die Verweilzeit im Allgemeinen im Bereich von 0,5 bis 50 h, bevorzugt im Bereich von 1 bis 30 h und besonders bevorzugt im Bereich von 1,5 bis 25 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Verweilzeit im Allgemeinen im Bereich von 0,1 bis 20 h, bevorzugt im Bereich von 0,2 bis 15 h und besonders bevorzugt im Bereich von 0,3 bis 10 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Das Gemisch, das aus dem ersten Rohrreaktor erhalten wird, enthält Cyclododecanon in einem Anteil, bezogen auf den Gesamtgehalt des Gemisches an C₁₂-Komponenten, bevorzugt im Bereich von 50 bis 99,9 Gew.-% und besonders bevorzugt im Bereich von 70 bis 99,5 Gew.-%. Dieses Gemisch wird, gegebenenfalls nach mindestens einer geeigneten Zwischenbehandlung, dem zweiten Rohrreaktor zugeführt. Das Gemisch, das aus dem zweiten Rohrreaktor erhalten wird, enthält Cyclododecanon, in einem Anteil bevorzugt im Bereich von mindestens 99,5 %, weiter bevorzugt im Bereich von 99,9 % und insbesondere von 99,99 Gew.-%., besonders bevorzugt im Bereich von mindestens 99,9 % und insbesondere bevorzugt von mindestens 99,99 Gew.-%.

Der Wasserstoffdruck liegt bei der erfindungsgemäßen Hydrierung im Allgemeinen im Bereich von 1 bis 325 bar, bevorzugt im Bereich von 1,5 bis 200 bar, weiter bevorzugt im Bereich von 2 bis 100 bar und insbesondere bevorzugt im Bereich von 2,5 bis 50 bar.

Die Hydriertemperatur liegt im Allgemeinen im Bereich von 0 bis 250 °C, bevorzugt im Bereich von 20 bis 200 °C, beispielsweise im Bereich von 30 bis 180°C, weiter bevorzugt im Bereich von 30 bis 150 °C, besonders bevorzugt im Bereich von 40 bis 170°C und insbesondere bevorzugt im Bereich von 40 bis 140 °C.

Demgemäß betrifft die vorliegende Erfindung auch ein wie oben beschriebenes Verfahren, das dadurch gekennzeichnet ist, dass die Hydrierung in Anwesenheit eines Hydrierkatalysators, bevorzugt eines heterogenen Hydrierkatalysators, bei einer Temperatur im Bereich von 0 bis 250 °C und einem Druck im Bereich von 1 bis 325 bar durchgeführt wird.

Im Rahmen der erfindungsgemäßen Hydrierung kann mindestens ein geeignetes Lösungsmittel oder Verdünnungsmittel eingesetzt werden. Als solche sind unter anderem Cyclododecanon oder Cyclododecan sowie grundsätzlich sämtliche Lösungs- und Verdünnungsmittel zu nennen, die unter den Hydrierbedingungen nicht hydriert oder anderweitig umgesetzt werden.

Gemäß einer bevorzugten Ausführungsform des erfindungsgemäßen Verfahrens wird die Hydrierung ohne Zusatz eines Lösungs- oder Verdünnungsmittels durchgeführt.

Aus der erfindungsgemäßen Hydrierung wird im Allgemeinen ein Gemisch erhalten, das neben Cyclododecanon gegebenenfalls mindestens ein Nebenprodukt und/oder mindestens ein nicht umgesetztes Edukt und/oder mindestens eine weitere Verbindung enthält, die über beispielsweise ein Gemisch, enthaltend Edukt, der Hydrierung zugeführt wurde. Aus diesem Gemisch kann das Cyclododecanon über mindestens eine geeignete Methode wie beispielsweise bevorzugt über mindestens eine Destillation abgetrennt werden.

Das oben beschriebene erfindungsgemäße Verfahren zur Herstellung von Cyclododecanon bietet unter anderem den Vorteil, dass Cyclododecanon und auch Cyclododeca-4,8-dienon in wenigen Schritten und gleichzeitig mit hoher Selektivität erhalten werden. Ein weiterer erheblicher Vorteil ist die Tatsache, dass als ein Edukt für das erfindungsgemäße Verfahren Distickstoffmonoxid enthaltende Abgase aus bevorzugt industriellen Anlagen eingesetzt werden können, die einerseits ohne großen Aufwand verfügbar sind, die andererseits die Integration des erfindungsgemäßen Verfahrens in einen bestehenden Anlagenverbund ermöglichen, wodurch der Transportweg für das Edukt minimal gehalten werden kann, und die weiterhin, als potentielle Klimagase, nicht einer besonderen Behandlung zur Entsorgung zugeführt werden müssen, sondern direkt in ein Wertprodukt fließen.

Das erfindungsgemäß insbesondere bevorzugt erhaltene und gegebenenfalls aus dem Produktgemisch abgetrennte Cyclododecanon kann beispielsweise besonders bevorzugt zur Herstellung von Dodecandicarbonsäure und/oder Laurinlactam und/oder daraus abgeleiteten Polymeren wie beispielsweise Polyamiden wie Nylon 12 oder Nylon 6.12 eingesetzt werden.

Die vorliegende Erfindung wird durch die folgenden Beispiele illustriert.

### Beispiele

### Beispiel 1 : Oxidation von cis,trans,trans-1,5,9-Cyclododecatrien mit N₂O

In einem 250 ml Autoklav wurden 92,67 g (0,56 mol) cis,trans,trans-1,5,9-Cyclododecatrien (ca. 98 %ig, kommerziell erhältlich von der Fa. DuPont) vorgelegt. Der Autoklav wurde dann verschlossen und mit N₂ gespült. Anschließend wurde der Autoklav mit N₂O bis auf 30 bar aufgepresst. Die Temperatur wurde dann auf 225 °C erhöht (maximaler Druck während der Reaktion: 55 bar). Nach 50 h Reaktionszeit wurde der Autoklav abgekühlt und entspannt. Das Produkt (96 g) wurde mittels quantitativer GC und GC/MS analysiert. Der Umsatz an cis,trans,trans-1,5,9-Cyclododecatrien betrug 45 %. Die Selektivität zu Cyclododeca-4,8-dienon als Isomerengemisch betrug 92 % (molare Isomerenverhältnisse cis,trans : trans;cis : trans, trans gleich 1 : 1 : 0,1). Es bildeten sich lediglich geringe Mengen von Diketocyclododecenen, von denen mit GC/MS insgesamt fünf verschiedene Isomere nachgewiesen wurden, und Spuren von Dodeca-4,8,11-trienal (als Isomerengemisch).

### Beispiel 2 : Oxidation von cis,trans,trans-1,5,9-Cyclododecatrien mit N₂O

In einem 250 ml Autoklav wurden 94,30 g (0,57 mol) cis,trans,trans-1,5,9-Cyclododecatrien (ca. 98 %ig, kommerziell erhältlich von der Fa. DuPont) vorgelegt. Der Autoklav wurde dann verschlossen und mit N₂ gespült. Anschließend wurde der Autoklav mit N₂O bis auf 50 bar aufgepresst. Die Temperatur wurde dann auf 200 °C erhöht (maximaler Druck während der Reaktion: 81 bar). Nach 10 h Reaktionszeit wurde der Autoklav abgekühlt und entspannt. Das Produkt (102,2 g) wurde mittels quantitativer GC und GC/MS analysiert. Der Umsatz an cis,trans,trans-1,5,9-Cyclododecatrien betrug 35 %. Die Selektivität zu Cyclododeca-4,8-dienon als Isomerengemisch betrug 92 % (molare Isomerenverhältnisse cis,trans : trans,cis : trans,trans gleich 1 : 1 : 0,08). Es bildeten sich lediglich geringe Mengen von Diketocyclododecenen, von denen mit GC/MS insgesamt fünf verschiedene Isomere nachgewiesen wurden, und Spuren von Dodeca-4,8,11-trienal (als Isomerengemisch).

### Beispiel 3 : Oxidation von cis,trans,trans-1,5,9-Cyclododecatrien mit N₂O

In einem 250 ml Autoklav wurden 91,5 g (0,56 mol) cis,trans,trans-1,5,9-Cyclododecatrien (ca. 98 %ig, kommerziell erhältlich von der Fa. DuPont) vorgelegt. Der Autoklav wurde dann verschlossen und mit N₂ gespült. Anschließend wurde der Autoklav mit N₂O bis auf 50 bar aufgepresst. Die Temperatur wurde dann auf 200°C erhöht (maximaler Druck während der Reaktion: 92 bar). Nach 5 h Reaktionszeit wurde der Autoklav abgekühlt und entspannt. Das Produkt (99,4 g) wurde mittels quantitativer GC und GC/MS analysiert. Der Umsatz an cis,trans,trans-1,5,9-Cyclododecatrien betrug 30%. Die Selektivität zu Cyclododeca-4,8-dienon als Isomerengemisch betrug 95 % (molare Isomerenverhältnisse cis,trans : trans,cis : trans,trans gleich 1 : 1 : 0,08). Es bildeten sich lediglich geringe Mengen von Diketocyclododecenen, von denen mit GC/MS insgesamt fünf verschiedene Isomere nachgewiesen wurden, und Spuren von Dodeca-4,8,11-trienal (als Isomerengemisch).

### Beispiel 4: Oxidation von zurückgewonnenem cis,trans,trans-1,5,9-Cyclododecatrien mit N₂O

Die Austräge aus den Beispielen 1 bis 3 wurden gesammelt und im Vakuum destilliert. Die erste Fraktion, die bei 111 °C (20 mbar) siedete (T_{Kopf}), bestand im Wesentlichen aus cis,trans,trans-1,5,9-Cyclododecatrien. Die zweite Fraktion, die bei 140 °C (20 mbar) siedete (T_{Kopf}), bestand im Wesentlichen aus Cyclododeca-4,8-dienon. Fraktion 1 wurde unter den Bedingungen von Beispiel 3 oxidiert. Sowohl Umsatz als auch Selektivität blieben gegenüber Beispiel 3 unverändert.

### Beispiel 5 : Hydrierung von Cyclododeca-4,8-dienon zu Cyclododecanon

50 g Cyclododeca-4,8-dienon (Isomerengemisch, Fraktion 2 aus Beispiel 4) und 1 g Pd/C-Katalysator (10 Gew.-% Pd, kommerziell erhältlich von der Fa. Degussa unter der Produktnummer E 101 N/D) wurden in einen 100 ml-Autoklaven eingefüllt. Unter Rühren wurden 5 bar Wasserstoff aufgepresst. Bei 50 °C Reaktionstemperatur wurde so lange (10 h) Wasserstoff nachgeliefert, bis die Wasserstoffaufnahme zum Erliegen kam. Nach Abkühlen, Entspannen und Abfiltrieren des Katalysators wurde der Austrag gaschromatographisch analysiert. Der Umsatz an Cyclododeca-4,8-dienon war quantitativ. Es wurde Cyclododecanon in im Wesentlichen quantitativer Ausbeute (> 98 %) erhalten. Als Verunreinigungen konnten lediglich Spuren von Cyclododecan, Cyclododecenon und Dodecanal mit GC/MS nachgewiesen werden.

### Beispiel 6 : Hydrierung von Cyclododeca-4,8-dienon zu Cyclododecanon

In einem 30 ml-Rohrreaktor mit Flüssigkeitsumlauf wurden 28 ml Pd/C-Katalysator (5 Gew.-% Pd, kommerziell erhältlich von der Fa. Degussa unter der Produktnummer E 101 ND/W) eingefüllt. Nach Spülen mit Stickstoff wurden 10 bar Wasserstoffdruck eingestellt, auf 60 °C aufgeheizt und mittels Zulaufpumpe 150 ml Methanol (als Anfahrhilfe) in das System gepumpt. Anschließend wurde die Umlaufpumpe in Betrieb genommen (Umlauf: in etwa 100 ml/h) und danach der Zulauf auf 10 ml/h Cyclododeca-4,8-dienon umgestellt (Rieselfahrweise). Im Austrag fanden sich nach 24 h neben 1 Gew.-% Methanol ungefähr 98 Gew.-% Cyclododecanon und noch in etwa 1 Gew.-% ungesättigte Verbindungen. Nach weiteren 24 h und einer Temperaturerhöhung auf 70 °C wurden 99,5 Gew.-% Cyclododecanon sowie 0,5 Gew.-% ungesättigte Verbindungen gefunden.

Anschließend wurde die Anlage so umgebaut, dass zusätzlich noch ein Rohrreaktor (Nachreaktor, 10 ml Inhalt) installiert wurde, der ebenfalls bei 70 °C betrieben wurde. Im Austrag des Nachreaktors wurden keine ungesättigten Verbindungen mehr gefunden und die Ausbeute an Cyclododecanon war im Wesentlichen quantitativ (> 98 %).

### Beispiel 7 :

Aus entsprechenden Vorlagebehältem werden 2000 g/h *cis,trans,trans-1,5,9-*Cyclododecatrien und 68 g/h flüssiges N₂O über einen statischen Mischer in einen Rohrreaktor (Doppelmantelrohr, gewickelt, Durchmesser innen 6mm, Länge 36m) gepumpt. Das Rohr wird mittels Wärmeträgeröl auf 280°C thermostatisiert. Der Reaktionsinnendruck wird auf 100 bar geregelt. Nach Passieren der Reaktionszone wird das Reaktionsgemisch in zwei Flashbehältem zunächst auf 3 bar und anschließend auf 60 mbar entspannt, um den gebildeten N₂ und unumgesetztes N₂O abzuführen.

Das flüssige Produkt wird dann in einer Kolonne mit mindestens 7 theoretischen Trennstufen bei 60 mbar destilliert (T_{Sumpf}=170°C, T_{Kopf}130°C). Als Kopfprodukt erhält man unumgesetztes *cis, trans, trans*-1,5,9-Cyclododecatrien, das wieder in die Reaktion zurückgeführt wird. Der Sumpfaustrag, der hauptsächlich Cyclododeca-4,8-dienon enthält, wird in eine weitere Kolonne mit mindestens 12 theoretischen Trennstufen überführt und bei 45 mbar destilliert. Hier wird das Cyclododeca-4,8-dienon als Isomerengemisch über Kopf abdestilliert (T_{sumpf}=193°C, T_{Kopf}=60°C).

Es werden im Mittel 209 g/h Cyclododeca-4,8-dienon erhalten. Die Selektivität zu Cyclododeca-4,8-dienon beträgt 95% (bezogen auf *cis,trans,trans*-1,5,9-Cyclododecatrien).

## Patentansprüche

1. Verfahren zur Herstellung eines Ketons, umfassend die Umsetzung von 1,5,9-Cyclododecatrien mit Distickstoffmonoxid unter Erhalt von Cyclododeca**-4,8-**dienon.

2. Verfahren nach Anspruch 1, **dadurch gekennzeichnet, dass** als Distickstoffinonoxidquelle mindestens ein Distickstoffmonoxid enthaltendes Abgas mindestens eines industriellen Verfahrens dient.

3. Verfahren nach Anspruch 2, **dadurch gekennzeichnet, dass** die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

4. Verfahren nach einem der Ansprüche 1 bis 3, **dadurch gekennzeichnet, dass 1,5,9-**Cyclododecatrien mit einem Gasgemisch, enthaltend 20 bis 99,9 Gew.-% Distickstoffmonoxid, bezogen auf das Gesamtgewicht des Gasgemisches, umgesetzt wird.

5. Verfahren nach einem der Ansprüche 1 bis 4, **dadurch gekennzeichnet, dass** Distickstoffmonoxid oder das Gasgemisch enthaltend Distickstoffmonoxid in flüssiger Form eingesetzt wird.

6. Verfahren nach einem der Ansprüche 1 bis 5, **dadurch gekennzeichnet, dass** die Umsetzung bei einer Temperatur im Bereich von 140 bis 350 °C und einem Druck im Bereich von 1 bis 1000 bar durchgeführt wird.

7. Verfahren nach einem der Ansprüche 1 bis 6, **dadurch gekennzeichnet, dass** die Umsetzung bei einer Selektivität, bezogen auf **Cyclododeca-4,8-dienon**, von mindestens 90 % einen Umsatz von **1,5,9**-Cyclododecatrien im Bereich von 1 bis 80 % aufweist.

8. Verfahren nach einem der Ansprüche 1 bis 7, **dadurch gekennzeichnet, dass** als 1,5,9-Cyclododecatrien cis,trans,trans-1,5,9-Cyclododecatrien eingesetzt und mit Distickstoffmonoxid zu Cyclododeca-4,8-dienon umgesetzt wird.

9. Verfahren zur Herstellung von Cyclododecanon **gemäß Anspruch 1**, umfassend die Schritte (I) und (II)
(I) Umsetzung von 1,5,9-Cyclododecatrien mit Distickstoffmonoxid unter Erhalt von **Cyclododeca-4,8-dienon;**
(II) Hydrierung des gemäß (I) erhaltenen **Cyclododeca-4,8-dienons** unter Erhalt von Cyclododecanon.

10. Verfahren nach Anspruch 9, **dadurch gekennzeichnet, dass** als Distickstoffmonoxidquelle mindestens ein Distickstoffmonoxid enthaltendes Abgas mindestens eines industriellen Verfahrens dient.

11. Verfahren nach Anspruch 10, **dadurch gekennzeichnet, dass** die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

12. Verfahren nach einem der Ansprüche 9 bis 11, **dadurch gekennzeichnet, dass 1,5,9-**Cyclododecatrien mit einem Gasgemisch, enthaltend 20 bis 99,9 Gew.-% Distickstoffmonoxid, bezogen auf das Gesamtgewicht des Gasgemisches, umgesetzt wird.

13. Verfahren nach einem der Ansprüche 9 bis 12, **dadurch gekennzeichnet, dass** Distickstoffmonoxid oder das Gasgemisch enthaltend Distickstoffmonoxid in flüssiger Form eingesetzt wird.

14. Verfahren nach einem der Ansprüche 9 bis 13, **dadurch gekennzeichnet, dass** die Umsetzung gemäß (I) bei einer Temperatur im Bereich von 140 bis 350 °C und einem Druck im Bereich von 1 bis 1000 bar durchgeführt wird.

15. Verfahren nach einem der Ansprüche 9 bis 14, **dadurch gekennzeichnet, dass** die Umsetzung gemäß (I) bei einer Selektivität, bezogen auf **Cyclododeca-4,8-dienon**, von mindestens 90 % einen Umsatz von **1,5,9**-Cyclododecatrien im Bereich von 1 bis 80 % aufweist.

16. Verfahren nach einem der Ansprüche 9 bis 15, **dadurch gekennzeichnet, dass** als 1,5,9-Cyclododecatrien cis,trans,trans-1,5,9-Cyclododecatrien eingesetzt und gemäß (I) mit Distickstoffmonoxid zu Cyclododeca-4,8-dienon umgesetzt wird.

17. Verfahren nach einem der Ansprüche 9 bis 16, **dadurch gekennzeichnet, dass** die Hydrierung gemäß (II) in Anwesenheit eines heterogenen Hydrierkatalysators bei einer Temperatur im Bereich von 0 bis 250 °C und einem Druck im Bereich von 1 bis 325 bar durchgeführt wird.

## Claims

1. A process for preparing a ketone, comprising the reaction of 1,5,9-cyclododecatriene with dinitrogen monoxide to obtain cyclododeca-4,8-dienone.

2. The process according to claim 1, wherein the dinitrogen monoxide source is at least one dinitrogen monoxide-comprising offgas from at least one industrial process.

3. The process according to claim 2, wherein the dinitrogen monoxide source is the offgas of an adipic acid plant and/or of a dodecanedioic acid plant and/or of a hydroxylamine plant and/or of a nitric acid plant operated with the offgas of an adipic acid plant and/or of a dodecanedioic acid plant and/or of a hydroxylamine plant.

4. The process according to any of claims 1 to 3, wherein 1,5,9-cyclododecatriene is reacted with a gas mixture comprising from 20 to 99.9% by weight of dinitrogen monoxide, based on the total weight of the gas mixture.

5. The process according to any of claims 1 to 4, wherein dinitrogen monoxide or the gas mixture comprising dinitrogen monoxide is used in liquid form.

6. The process according to any of claims 1 to 5, wherein the reaction is carried out at a temperature in the range from 140 to 350°C and a pressure in the range from 1 to 1000 bar.

7. The process according to any of claims 1 to 6, wherein the reaction has a conversion of 1, 5, 9-cyclododecatriene in the range from 1 to 80% at a selectivity based on cyclododeca-4,8-dienone of at least 90%.

8. The process according to any of claims 1 to 7, wherein the 1, 5, 9-cyclododecatriene used is cis,trans,trans-1,5,9-cyclododecatriene and is reacted with dinitrogen monoxide to give cyclododeca-4,8-dienone.

9. A process for preparing cyclododecanone according to claim 1, comprising the steps (I) and (II)
(I) reacting 1, 5, 9-cyclododecatriene with dinitrogen monoxide to obtain cyclododeca-4,8-dienone;
(II) hydrogenating the cyclododeca-4,8-dienone obtained in (I) to obtain cyclododecanone.

10. The process according to claim 9, wherein the dinitrogen monoxide source is at least one dinitrogen monoxide-comprising offgas from at least one industrial process.

11. The process according to claim 10, wherein the dinitrogen monoxide source is the offgas of an adipic acid plant and/or of a dodecanedioic acid plant and/or of a hydroxylamine plant and/or of a nitric acid plant operated with the offgas of an adipic acid plant and/or of a dodecanedioic acid plant and/or of a hydroxylamine plant.

12. The process according to any of claims 9 to 11, wherein 1, 5, 9-cyclododecatriene is reacted with a gas mixture comprising from 20 to 99.9% by weight of dinitrogen monoxide, based on the total weight of the gas mixture.

13. The process according to any of claims 9 to 12, wherein dinitrogen monoxide or the gas mixture comprising dinitrogen monoxide is used in liquid form.

14. The process according to any of claims 9 to 13, wherein the reaction in (I) is carried out at a temperature in the range from 140 to 350°C and a pressure in the range from 1 to 1000 bar.

15. The process according to any of claims 9 to 14, wherein the reaction in (I) has a conversion of 1, 5, 9-cyclododecatriene in the range from 1 to 80% at a selectivity based on cyclododeca-4,8-dienone of at least 90%.

16. The process according to any of claims 9 to 15, wherein the 1,5, 9-cyclododecatriene used is cis,trans,trans-1,5,9-cyclododecatriene and is reacted in (1) with dinitrogen monoxide to give cyclododeca-4,8-dienone.

17. The process according to any of claims 9 to 16, wherein the hydrogenation in (II) is carried out in the presence of a heterogeneous hydrogenation catalyst at a temperature in the range from 0 to 250°C and a pressure in the range from 1 to 325 bar.

## Revendications

1. Procédé pour la préparation d'une cétone, comprenant la mise en réaction de 1,5,9-cyclododécatriène avec du protoxyde d'azote, avec obtention de cyclododéca-4,8-diénone.

2. Procédé selon la revendication 1, **caractérisé en ce qu'**on utilise comme source de protoxyde d'azote au moins un gaz rejeté d'au moins un processus industriel, contenant du protoxyde d'azote.

3. Procédé selon la revendication 2, **caractérisé en ce que** la source de protoxyde d'azote est le gaz rejeté d'une unité d'acide adipique et/ou d'une unité d'acide dodécanedioïque et/ou d'une unité d'hydroxylamine et/ou d'une unité d'acide nitrique fonctionnant avec le gaz rejeté d'une unité d'acide adipique et/ou d'une unité d'acide dodécanedioïque et/ou d'une unité d'hydroxylamine.

4. Procédé selon l'une quelconque des revendications 1 à 3, **caractérisé en ce qu'**on fait réagir le **1,5,9**-cyclododécatriène avec un mélange de gaz contenant 20 à 99,9 % en poids de protoxyde d'azote, par rapport au poids total du mélange de gaz.

5. Procédé selon l'une quelconque des revendications 1 à 4, **caractérisé en ce que** le protoxyde d'azote ou le mélange de gaz contenant du protoxyde d'azote est utilisé sous forme liquide.

6. Procédé selon l'une quelconque des revendications 1 à 5, **caractérisé en ce que** la réaction est effectuée à une température dans la plage de 140 à 350 °C et sous une pression dans la plage de 1 à 1 000 bars.

7. Procédé selon l'une quelconque des revendications 1 à 6, **caractérisé en ce que** la réaction, avec une sélectivité, rapportée à la **cyclododéca-4,8-diénone**, d'au moins 90 %, présente un degré de conversion du **1,5,9**-cyclododécatriène dans la plage de 1 à 80 %.

8. Procédé selon l'une quelconque des revendications 1 à 7, **caractérisé en ce qu'**on utilise en tant que 1,5,9-cyclododécatriène du *cis,trans,trans-*1,5,9-cyclododécatriène et on le convertit, avec du protoxyde d'azote, en cyclododéca-4,8-diénone.

9. Procédé pour la préparation de cyclododécanone **selon la revendication 1**, comprenant les étapes (I) et (II)
(I) mise en réaction de 1,5,9-cyclododécatriène avec du protoxyde d'azote, avec obtention de **cyclododéca-4,8-diénone ;**
(II) hydrogénation de la **cyclododéca-4,8-diénone** obtenue selon (I), avec obtention de cyclododécanone.

10. Procédé selon la revendication 9, **caractérisé en ce qu'**on utilise comme source de protoxyde d'azote au moins un gaz rejeté d'au moins un processus industriel, contenant du protoxyde d'azote.

11. Procédé selon la revendication 10, **caractérisé en ce que** la source de protoxyde d'azote est le gaz rejeté d'une unité d'acide adipique et/ou d'une unité d'acide dodécanedioïque et/ou d'une unité d'hydroxylamine et/ou d'une unité d'acide nitrique fonctionnant avec le gaz rejeté d'une unité d'acide adipique et/ou d'une unité d'acide dodécanedioïque et/ou d'une unité d'hydroxylamine.

12. Procédé selon l'une quelconque des revendications 9 à 11, **caractérisé en ce qu'**on fait réagir le **1,5,9**-cyclododécatriène avec un mélange de gaz contenant 20 à 99,9 % en poids de protoxyde d'azote, par rapport au poids total du mélange de gaz.

13. Procédé selon l'une quelconque des revendications 9 à 12, **caractérisé en ce que** le protoxyde d'azote ou le mélange de gaz contenant du protoxyde d'azote est utilisé sous forme liquide.

14. Procédé selon l'une quelconque des revendications 9 à 13, **caractérisé en ce que** la réaction selon (I) est effectuée à une température dans la plage de 140 à 350 °C et sous une pression dans la plage de 1 à 1 000 bars.

15. Procédé selon l'une quelconque des revendications 9 à 14, **caractérisé en ce que** la réaction selon (I), avec une sélectivité, rapportée à la **cyclododéca-4,8-diénone**, d'au moins 90 %, présente un degré de conversion du **1,5,9**-cyclododécatriène dans la plage de 1 à 80 %.

16. Procédé selon l'une quelconque des revendications 9 à 15, **caractérisé en ce qu'**on utilise en tant que 1,5,9-cyclododécatriène du *cis,trans,trans-*1,5,9-cyclododécatriène et on le convertit selon (I), avec du protoxyde d'azote, en cyclododéca-4,8-diénone.

17. Procédé selon l'une quelconque des revendications 9 à 16, **caractérisé en ce que** l'hydrogénation selon (II) est effectuée en présence d'un catalyseur d'hydrogénation hétérogène, à une température dans la plage de 0 à 250 °C et sous une pression dans la plage de 1 à 325 bars.
